# EUROPÄISCHE PATENTANMELDUNG

(11) **EP 1 228 698 A1**
(43) Veröffentlichungstag der Anmeldung: **07.08.2002**
(21) Anmeldenummer: 02001068.2
(22) Anmeldetag: 22.01.2002
(51) Int. Cl.: A23K 1/16, A23K 1/18

(54) **Sorbinsäure als wachstumsstabilisierender Zusatz zu Futtermitteln für die Nutztieraufzucht**

(30) Priorität: 02.02.2001 DE 10105308
(71) Anmelder: Nutrinova Nutrition Specialties & Food Ingredients GmbH, 65929 Frankfurt am Main (DE)
(72) Erfinder: Raczek, Nico, Dr., 65779 Kelkheim (DE)
(74) Vertreter: Schweitzer, Klaus, Dr.

(57) **Zusammenfassung**

Die vorliegende Erfindung betrifft die Verwendung von Sorbinsäure, bevorzugt in einer Konzentration von >0 bis <1,2 Gew.-% (bezogen auf das Futtermittel), mit wachstumsstabilisierendem Effekt. Die Futtermittel können zur Leistungsförderung in der Nutztieraufzucht eingesetzt werden.

## Beschreibung

Die Erfindung betrifft die Verwendung von Sorbinsäure als wachstumsstabilisierender Zusatz zu Futtermitteln bevorzugt in Konzentrationen von >0 bis < 1,2 Gew.-% (bezogen auf das Futtermittel).

Im Bereich der Tierfutter werden häufig Antibiotika als Leistungsförderer verwendet. Die Anwendung von Antibiotika in diesem Bereich steht im Verdacht für Gefahren, welche von resistenten Bakterien ausgehen, die auch die menschliche Gesundheit langfristig gefährden können, verantwortlich zu sein. Daher müssen gesundheitlich weniger bedenkliche Produkte für diesen Einsatzzweck gesucht werden. So werden auch in anderen Bereichen zunehmend physiologisch und epidemiologisch gesundheitlich bedenkliche oder aber für die Umwelt schädliche Substanzen, wie beispielsweise Antibiotika, formaldehydabspaltende Stoffe, halogenierte Substanzen, u.v.a.m. beispielsweise in Lebensmitteln, Futtermitteln, Haustierfutter, Silagen, Trester, oder anderen Abfällen aus der Lebensmittelindustrie, durch weniger bedenkliche Stoffe ersetzt. Der Zweck dieser Stoffe ist zum einen auf die Werterhaltung des eigentlichen Produktes gerichtet. Zum anderen aber soll auch deren hygienischer Zustand verbessert werden, bzw. eine verlängerte Haltbarkeit erzielt werden.

Es ist bekannt, dass Sorbinsäure zur Konservierung von Futtermitteln eingesetzt werden kann. Sorbinsäure (trans,trans-2,4-Hexadiensäure) ist eine farblose, feste Verbindung, die sich nur wenig in kaltem Wasser löst und weltweit als Konservierungsstoff verwendet wird. Das Wirkprinzip wird durch die Sorbinsäure in undissoziierter Form bestimmt. Die beste Wirkung entfaltet Sorbinsäure daher im sauren pH-Bereich. Sorbinsäure und ihre Salze besitzen eine sehr gute mikrobiostatische, antimykotische Wirkung. Gleichzeitig ist Sorbinsäure als ungesättigte Fettsäure praktisch ungiftig, was sehr umfangreiche Daten belegen, und durch die jahrzehntelange Anwendung dieser Säure im Lebensmittelbereich, in Tierfuttern u.a. belegt ist.

Es wurden früher Fütterungsversuche vor allem mit Ferkeln durchgeführt, die belegten, dass verschiedene organische Säuren, wie Zitronensäure, Fumarsäure oder Ameisensäure in der Lage sind, die tierischen Leistungen in positiver Weise zu beeinflussen, wenn sie in der optimalen Dosierung dem Ferkelfutter beigemischt werden (Zbl. Hyg. 191, 265-276, Kirchgessner und Roth, 1991; Journal of Animal and Feed Sciences, 7, 1998, 25 - 33, Roth und Kirchgessner). Diese Säuren wirken aber korrosiv und verursachen auf Grund ihrer Flüchtigkeit z. T. Geruchsbelästigungen und erfordern besondere Vorsicht im Umgang, wenn das Risiko einer aus Sicht des Gesundheitsschutzes unerwünschten inhalatorischen Aufnahme vermieden werden soll.

In jüngster Zeit konnte auch gezeigt werden, daß die Sorbinsäure in hohen Konzentrationen (1,2 - 2,4 % Sorbinsäure, bezogen auf das Futtermittel) eine nutritive Wirksamkeit für Aufzuchtferkel besitzt (J. Anim. Physiol. a. Anim. Nutr. 74 (1995), 235 - 242, Kirchgessner et al.). Auf der 6. Tagung Schweine- und Geflügelernährung (Tagungsband, S. 60, 61, J. Rühle et al., 'Zur Wirkung von Ameisen-, Milch- und Sorbinsäure auf einige Leistungs- und Stoffwechselkenndaten beim Absetzferkel') wurde über die Wirkung unter anderem von Sorbinsäure als Leistungsförderer bei der Ferkelaufzucht berichtet. Im Vergleich zu Ameisen- und Milchsäure wurde bei Sorbinsäure die beste ergotrope Wirkung erreicht. Die Konzentration der Sorbinsäure pro kg Futtermittel betrug bei diesen Untersuchungen 0,185 Mol/kg (ca. 2,1 Gew.-%). In Kraftfutter / Feed Magazine 2/99, S. 49ff (M. Freitag et al.) wird Sorbinsäure als leistungsfördernder Futtermittelzusatz 'im mittleren Konzentrationsbereich' beschrieben; bekannt sind Konzentrationsbereiche von 1,2 bis 2,4 Gew.-% Futtermittel (s. z. B. J. Anim. Physiol. a. Anim. Nutr. 74 (1995), 235 - 242, Kirchgessner et al.).

In der WO 00/36928 werden leistungsfördernde Futtermittelzusätze beschrieben, die C₆-C₁₀ Carbonsäuren oder Carbonsäuresalze enthalten. Diese Zusätze sind in den Futtermitteln in Mengen von 10 - 30 Gew.-% enthalten. Ungesättigte oder gar mehrfach ungesättigte Carbonsäuren sind dort nicht beschrieben, erst recht keine Sorbinsäure.

Sorbinsäure als aliphatische ungesättigte Carbonsäure zeigt eine erstaunlich hohe Lagerstabilität und greift Metalle kaum an und verursacht in praktischen Anwendungen kaum Geruchsbelästigungen, so dass Sorbinsäure vorteilhafte chemisch-physikalische Eigenschaften zur Verarbeitung in genanntem Bereich aufweist. Diese ist außerdem verbunden mit guten Eigenschaften in der Handhabung. Sorbinsäure ist deshalb ein idealer Zusatzstoff zu Futtermitteln. Nachteilig ist jedoch nach wie vor, dass der Einsatz von Sorbinsäure - insbesondere vor dem Hintergrund der hohen Konzentrationen - nicht wirtschaftlich ist. Es bestand das Bedürfnis nach einem preiswerten Futtermittel mit wachstumsstabilisierenden Zusätzen ohne die Nachteile der heute üblicherweise verwendeten Stoffe fort.

Überraschenderweise wurde gefunden, dass schon durch Zusatz geringerer Mengen an Sorbinsäure als bisher allgemein angenommen in der Nutztieraufzucht, insbesondere der Ferkelaufzucht eine deutliche und besonders wirtschaftliche Wachstumsverbesserung hinsichtlich Zuwachsrate und Futterverwertung erreicht werden kann. Sie ergibt sich bereits bei Zusätzen von >0 bis <1,2 Gew.-%, bezogen auf das Futter, insbesondere 0,5 - 1,0 Gew.-%, vorzugsweise von 0,625 - 0,875 Gew.-%.

Die Erfindung betrifft dementsprechend die Verwendung von Sorbinsäure als wachstumsstabilisierender Zusatz zu Futtermitteln, insbesondere in Konzentrationen von >0 bis < 1,2 Gew.-% (bezogen auf das Futtermittel). Die Erfindung betrifft ferner ein Futtermittel zur Erzielung eines wachstumsstabilisierenden Effekts, welches Sorbinsäure enthält, bevorzugt in einer Konzentration von >0 bis <1,2 Gew.-% (bezogen auf das Futtermittel) sowie Sorbinsäure enthaltende Produkte zur Herstellung von Futtermitteln.

Als Tierfuttermittel geeignet sind z. B. Grünfutter, Silagen, Trockengrünfutter, Wurzeln, Knollen, fleischige Früchte, Körner und Samen, Biertreber, Trester, Bierhefe, Destillationsschlempe, Müllereinebenerzeugnisse, Nebenerzeugnisse der Zucker-, Stärkeherstellung und Ölgewinnung und verschiedene Lebensmittelabfälle. Solchen Futtermitteln können bestimmte Futtermittelzusatzstoffe (z.B. Antioxidantien) oder Mischungen aus verschiedenen Substanzen (z.B. Mineralstoffmischungen, Vitaminmischungen) zu Verbesserung beigegeben werden. Spezielle Futtermittel werden auch für bestimmte Tierarten und deren Entwicklungsstadium angepaßt. Dies ist beispielsweise in der Ferkelaufzucht der Fall. Hier werden Saugferkelfutter und Ferkelaufzuchtfutter verwendet. Weiterhin eignen sich Futtermittel mit dem erfindungsgemäßen Zusatz an Sorbinsäure als Milchaustauscher bei der Frühentwöhnung von Sauglämmern oder Kälbern.

Sorbinsäure kann direkt dem Tierfuttermittel, einzelnen seiner Bestandteile oder anderen zugesetzten Stoffen wie Futtermittelzusatzstoffen oder aber über Vormischungen verschiedener Bestandteile dem eigentlichen Futtermittel zugegeben werden. Dazu gehören u. a. Mineralstoff-, Säure- und Vitaminmischungen, Geschmacksstoffpräparate, Ergänzungsfuttermittel, deren Mischungen sowie Mischungen derartiger Präparate mit Bestandteilen der Futtermittel. Sie können den Futtermitteln, einzelnen ihrer Bestandteile oder trocken dem Futter zugemischt werden, vor einer weiteren Verarbeitung (z.B. Extrusion) zugegeben werden bzw. im Gemisch dispergiert zudosiert werden. Wenn Sorbinsäure über einzelne Bestandteile des Futtermittels oder Vormischungen zugegeben wird, werden die Dosierungen dabei so gewählt, dass sich die erfindungsgemäßen Gehalte im Futtermittel ergeben.

Sorbinsäure liegt in fester Form vor. Sie kann dadurch problemlos in feste und pastöse Futtermittel eingearbeitet werden. Da die Löslichkeitsgrenze in wässrigen, auch nur leicht sauren Futtermitteln überschritten wird, wird zweckmäßigerweise Sorbinsäure geringer Korngröße eingesetzt, wobei mindestens 80 Gew.-% im Bereich unterhalb 555 µm, vorzugsweise sogar unterhalb 355 µm liegen sollten, um eine möglichst gleichmäßige Verteilung zu erreichen.

Die Erfindung wird nachfolgend anhand von Beispielen erläutert.

### Beispiel 1

Um die wachstumsstabilisierende Wirksamkeit der Sorbinsäure im erfindungsgemäßen Konzentrationsbereich zu untersuchen, wurden Fütterungsversuche mit je 48 Absetzferkeln in Einzelhaltung durchgeführt. Das Futter der vier Versuchsgruppen war isoenergetisch zusammengesetzt und wurde den Tieren zur freien Aufnahme vorgelegt. Im vorliegenden Versuch wurde die Wirksamkeit von Sorbinsäure im niedrigen Dosierungsbereich bei Ferkeln geprüft . Dazu wurden 0 (kein Zusatz); 0,1; 0,55 und 1 Gew.-% Sorbinsäure dem Futter zugesetzt. Die Ergebnisse dieses Versuches sind in Tabelle 1 zusammengefaßt. Der Zusatz von Sorbinsäure zum Futter zeigte bei den Tieren eine deutliche Wirkung. Insbesondere die Stufen 0,55 und 1 % Sorbinsäure steigerten die Wachstumsraten ausgehend von 491 g /Tag um 7 % bzw. 16 % (P < 0.05). Dabei nahmen die Tiere aufgrund des Sorbinsäurezusatzes um 7 % bzw. 14 % mehr Futter auf.

**Tabelle 1**

| Lebendgewichte, tägliche Zunahmen, Futterverzehr und Futterverwertung von Aufzuchtferkeln bei Zusatz von Sorbinsäure zum Futter | | | | |
|---|---|---|---|---|
| Zusatz berechnet als Gew.-% Sorbinsäure bezogen auf das Gesamtfutter; Tag 0-41 | - | 0,1 | 0,55 | 1,0 |
| Anfangsgewicht, kg | 7,60 | 7,60 | 7,60 | 7,60 |
| | ±0,82 | ±0,98 | ±1,04 | ±0,94 |
| Endgewicht, kg | 27,72^{ab} | 26,69^{b} | 29,16^{ab} | 30,96^{a} |
| | ±3,04 | ±3,00 | ±3,48 | ±3,35 |
| relativ | 100 | 96,3 | 105,2 | 111,7 |
| Zuwachsrate, g/d | 491^{b} | 466^{b} | 526^{ab} | 570^{a} |
| | ±72 | ±65 | ±65 | ±73 |
| relativ | 100 | 94,5 | 107,1 | 116,1 |
| Futterverzehr, g/d | 678^{ab} | 640^{b} | 729^{ab} | 775^{a} |
| | ±102 | ±96 | ±102 | ±93 |
| relativ | 100 | 94,4 | 107,5 | 114,3 |
| Futterverwertung | 1,38 | 1,38 | 1,38 | 1,36 |
| (g Futter/g Zuwachs) | ±0,04 | ±0,08 | ±0,06 | ±0,08 |
| relativ | 100 | 100 | 100 | 98,5 |

| | | | | |
|---|---|---|---|---|
| ^{a,b} Signifikant unterschiedliche Mittelwerte (p < 0.05) | | | | |

Werden die Ergebnisse identisch angelegter Versuche verglichen, so muss man außerdem überraschenderweise feststellen, dass insbesondere beim Zusatz von Sorbinsäure zu Saugferkelfutter (Prestarter) keine lineare Abhängigkeit in den Zuwachsraten und der Futterverwertung der Tieren gegeben ist: In der Literatur werden bei Einsatzkonzentrationen von 1,2 (109 %), 1,8 (117%) und 2,4 % (123%) Sorbinsäurezulage zum Futter die in Klammern genannten relativen Zunahmen erhalten (J. Anim. Physiol. a. Anim. Nutr. 74 (1995), 235 - 242, Kirchgessner et al.: ,Zur nutritiven Wirkung von Sorbinsäure in der Ferkelaufzucht'). Bei dem Einsatz von Sorbinsäure in den erfindungsgemäßen Konzentrationen hingegen, werden bereits bei einem Zusatz von 0,55 (115%) und 1,0 % (128%) Sorbinsäure die in Klammern genannten, wesentlich höheren Zuwachsraten erreicht.

Weiterhin wird beim Einsatz von Sorbinsäure in den erfindungsgemäßen Konzentrationen erstaunlicherweise eine bessere Futterverwertung durch die Tiere - verglichen mit höheren Konzentrationsbereichen - erreicht.

## Patentansprüche

1. Verwendung von Sorbinsäure als wachstumsstabilisierender Zusatz zu Futtermitteln.

2. Verwendung nach Anspruch 1, **dadurch gekennzeichnet, dass** die Sorbinsäure in Konzentrationen von >0 bis < 1,2 Gew.-% (bezogen auf das Futtermittel) eingesetzt wird.

3. Verwendung nach Anspruch 1, **dadurch gekennzeichnet, dass** die Konzentration der Sorbinsäure im Bereich von 0,5 bis 1,0 Gew.-% (bezogen auf das Futtermittel) liegt.

4. Verwendung nach Anspruch 1 in der Schweineaufzucht.

5. Verwendung nach Anspruch 1 in der Rinderaufzucht.

6. Verwendung nach Anspruch 1 in der Lämmeraufzucht.

7. Verwendung nach Anspruch 1 in der Geflügelaufzucht.

8. Verwendung nach Anspruch 1 in der Kaninchenaufzucht.
